(19) Europäisches Patentamt
European Patent Office
Office européen des brevets

(11) EP 3 545 894 A1

(12) EUROPEAN PATENT APPLICATION

(43) Date of publication:
02.10.2019 Bulletin 2019/40

(51) Int Cl.:
A61B 34/10 (2016.01)      A61B 34/20 (2016.01)
A61N 5/10 (2006.01)       A61B 90/00 (2016.01)

(21) Application number: 18164826.2

(22) Date of filing: 29.03.2018

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicant: Koninklijke Philips N.V.
5656 AE Eindhoven (NL)

(72) Inventors:
• KUSTRA, Jacek Lukasz
5656 AE Eindhoven (NL)

• MEHENDALE, Aditya
5656 AE Eindhoven (NL)
• HAUTVAST, Guillaume Leopold Theodorus
Frederik
5656 AE Eindhoven (NL)
• FRISSEN, Petrus Carolus Maria
5656 AE Eindhoven (NL)
• POTZE, Willem
5656 AE Eindhoven (NL)

(74) Representative: de Haan, Poul Erik et al
Philips International B.V.
Philips Intellectual Property & Standards
High Tech Campus 5
5656 AE Eindhoven (NL)

(54) MAGNETIC LOCALIZATION ARRANGEMENT FOR MEDICAL DEVICE

(57) The invention relates to a system and a method for localizing at least one medical device (1a-c) in a patient body (2). The at least one medical device (1a-c) comprises at least one marker (3a-c) which is capable of at least temporarily generating a magnetic field. Further, the system comprises magnetometers (7i) arranged outside the patient body (2) for measuring the magnetic field generated by the at least one marker (3a-c), and an evaluation unit (8) coupled to the magnetometers (7i) and configured to determine the position of the at least one medical device (1a-c) on the basis of the magnetic field measurement by the magnetometers (7i).

FIG. 1

**Description**

FIELD OF THE INVENTION

The invention relates to the localization of a medical device, particularly during an interventional procedure. More specifically, the invention is related to a system and a method for localizing at least one medical device in a patient body.

BACKGROUND OF THE INVENTION

In medical treatments of patients including interventional procedures, it is often necessary or at least helpful to localize the medical devices used in these procedures in order to assist the operator in navigating the medical devices and/or in order to further plan the treatment of the patient. Often it is also desirable to track the medical devices in real-time in such treatments.

Examples of such treatments include minimally invasive diagnostic or therapeutic treatments. In such procedures, the medical devices to be tracked include guidewires, catheters, needles and imaging devices, such as endoscopes or ultrasound transducers, for example. Another example of such treatments is high dose radiation (HDR) brachytherapy for treating cancer. In this kind of treatment, radiation sources are placed inside a patient body, which emit ionizing radiation to treat the surrounding tissue with the main goal to destroy cancer cells. The radiation sources are placed in the patient body by means of application catheters and these catheters have to be tracked in order to properly carry out the treatment.

Known techniques for localizing medical devices include image-based tracking on the basis of images of the region of the patient body including the medical devices to be tracked. In this respect, imaging modalities such as computed tomography (CT) imaging and magnetic resonance (MR) imaging allow for a relatively precise localization of medical devices, but require large equipment and usually do not allow for a real-time tracking. For an image-based real-time localization, x-ray fluoroscopy or ultrasound (US) imaging may be applied. However, the image quality provided by these imaging modalities is often not sufficient for a precise localization of the medical devices.

A further known localization technique is electromagnetic (EM) tracking. In this tracking technique, the medical devices move in magnetic fields generated by field generators and are equipped with miniaturized magnetic sensors. It is a drawback of this tracking technique that the magnetic sensors are active devices, which have to be electrically connected to a unit outside the patient body in order to supply the sensors with power and to process the signals provided by the sensors. This complicates the design of the medical devices to be tracked and their handling during an interventional procedure.

SUMMARY OF THE INVENTION

It is therefore an object of the present invention to allow for an easier localization of medical devices in a patient body.

In accordance with one aspect, the invention suggests a system for localizing at least one medical device in a patient body, the at least one medical device comprising at least one marker which is capable of at least temporarily generating a magnetic field. The system comprises magnetometers arranged outside the patient body for measuring the magnetic field generated by the at least one marker, and an evaluation unit coupled to the magnetometers and configured to determine the position of the at least one medical device on the basis of the magnetic field measurement by the magnetometers.

Since the at least one marker included in the at least one medical device is passive device generating a magnetic field, which is measured using magnetometers outside the patient body, the medical device can easily be localized. In particular, it is not necessary to integrate active devices and related connections to units outside the patient body into the medical device. Moreover, the at least one marker allows for an accurate localization of the at least one medical device.

In one embodiment, the system is configured for simultaneously localizing a plurality of medical devices, wherein each of the plurality of medical devices comprises one marker at a specific point thereof. In this embodiment, the determination of the positions of the medical devices particularly includes the determination of the positions of the specific points thereof, where the markers are positioned. The localization of such points, which can be carried out simultaneously for a plurality of medical devices in the system, is often sufficient and it is often not necessary to localize further points of the medical devices.

In a further embodiment, the system is configured for localizing one medical device, wherein the medical device comprises a plurality of markers and wherein the evaluation unit is configured to determine the positions of the medical device by determining positions of the markers and fitting a model of the medical device to the determined positions. This embodiment allows for determining the position and orientation of the entire magnetic device. Thus, this embodiment may be applied if a localization of a specific point is not sufficient for a medical device. The fitting of the model of the medical device to the determined positions of the markers in the embodiment, preferably includes a positioning of the model such that points thereof corresponding to the locations of the markers of the real device are placed at the determined positions of the markers.

In one embodiment, the at least one marker is configured to generate a magnetic field in response to a magnetic excitation by a magnetic excitation field. The magnetic field generated as a result of the magnetic excitation is also referred to as induced magnetic field herein. Compared with a permanently magnetized marker of a similar size, a marker generating an induced magnetic field has the advantage that it generates a stronger magnetic field, which can be measured more accurately by means of the magnetometers. Thus, it is possible to provide smaller markers which can be integrated into the medical device(s) more easily.

In a related embodiment, the at least one marker comprises a magnetizable material and the magnetic field is generated as a result of a magnetization of the magnetizable material by means of the magnetic excitation field. In further related embodiments, the at least one marker comprises an electrically conductive material or a resonant circuit and the magnetic field is generated as a result of an excitation by a time-varying magnetic excitation field. When the marker comprises an electrically conductive material, which is substantially not magnetizable, the magnetic field is particularly generated due to eddy currents induced in the electrically conductive material, which in turn generate the induced magnetic field.

In a related embodiment, the system further comprises at least one magnetic field generator for generating the magnetic excitation field. In a related embodiment, the magnetic field generator is configured to generate the magnetic excitation field with a varying strength and the magnetic field measurement is carried out at a time when the strength of the excitation magnetic field is minimal. The minimum strength of the magnetic excitation field may particularly be substantially zero. In general, the induced magnetic field to be measured interferes with the magnetic excitation field. However, by carrying out the magnetic field measurement when the strength of the magnetic excitation field is minimal, it is ensured that substantially only the induced magnetic field is measured. Thus, this embodiment allows for an easy discrimination between the induced magnetic field and the magnetic excitation field.

In one embodiment, the evaluation unit is configured to calculate values of the magnetic field generated by the markers at the locations of the magnetometers on the basis of estimated positions of the markers in a model calculation and to determine the positions of the markers by at least approximately minimizing a difference between the calculated values of the magnetic field and values measured by the magnetometers. In the model calculation, the markers may be regarded as magnetic dipoles and the magnetic field generated by the markers may be approximated as a superposition of magnetic fields of the magnetic dipoles in the model calculation.

In one embodiment, the medical devices are brachytherapy catheters for holding radiation sources during a brachytherapy treatment and each brachytherapy catheter may comprise one marker. In this embodiment, it is particularly possible to monitor the correct positioning of the brachytherapy catheters during the brachytherapy treatment. Hereby, a quality assurance for the brachytherapy treatment is possible.

A related embodiment includes that the system further comprises a planning unit configured to detect a deviation between the determined positions of the markers and desired positions thereof, which correspond to desired positions of the brachytherapy catheters during the brachytherapy treatment. The desired positions of the catheters may be specified in a treatment plan generated for the treatment. In addition, the treatment plan may specify the locations at which the radiation sources are to be held in the catheters during the treatment (these locations are also referred to as dwell points) and the durations for which the radiation sources are to be held at these locations (these durations are also referred to as dwell times).

The planning unit may particularly be configured to compare absolute values of the positions of the markers with absolute values of the desired positions. In an alternative embodiment, the planning unit is configured to compare relative positions of the markers computed based on the determined positions of the markers with desired relative positions of the markers determined based on the desired positions of the markers and to detect a deviation between the determined positions of the markers and desired positions thereof, if it determines that at least one of relative positions of the markers deviates from a correspond desired relative position. This embodiment has the advantage that no absolute positions reference is required.

In a further embodiment, the planning unit is configured to initiate an alarm routine when it detects a deviation between the determined positions of the markers and the desired positions thereof. In accordance with the alarm routine, a planner of the brachytherapy treatment may be notified and/or the treatment may be stopped automatically. When the planner is notified, he/she may further assess whether the treatment can be continued despite the deviation or whether a re-planning of the treatment is necessary, for example. In accordance with a further aspect, the invention suggests a method for localizing at least one medical device in a patient body, the at least one medical device comprising at least one marker which is capable of at least temporarily generating a magnetic field. The method comprises: (i) receiving measurement information from magnetometers arranged outside the patient body, the measurement information relating to measurements of the magnetic field generated by the at least one marker, and (ii) determining the position of the at least one medical device on the basis of the measurement information.

In accordance with a further aspect, the invention suggests a computer program comprising program code for instructing a computer device to perform the method when the program code is executed in the computer device.

It shall be understood that the system of claim 1, the method of claim 14 and the computer program of claim 15 have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

BRIEF DESCRIPTION OF THE DRAWINGS

In the following drawings:

Fig. 1 schematically and exemplarily shows components of a system for localizing a magnetic device,
Fig. 2 shows schematically and exemplarily one embodiment of an array of magnetometers,
Fig. 3a shows schematically and exemplarily an excitation magnetic field generated by a rectangular electromagnetic excitation coil, and
Fig. 3b shows schematically and exemplarily an induced magnetic field generated by an object in reaction to the excitation magnetic field.

DETAILED DESCRIPTION OF EMBODIMENTS

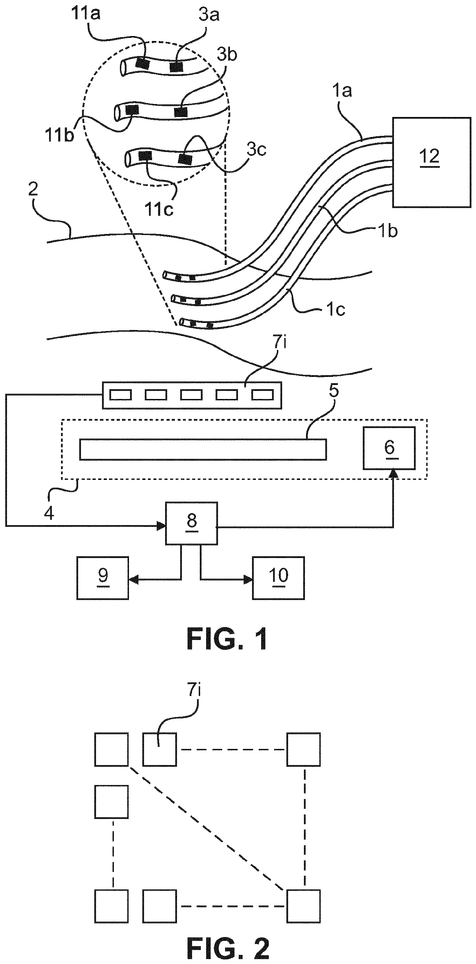

Fig. 1 schematically and exemplarily depicts a localization system for localizing one or more medical device(s) 1a-c in a body 2 of a patient. The medical device(s) 1a-c may be guidewires, catheters, needles or similar devices which may be used in an interventional procedure. In the system, the medical device(s) 1a-c is/are localized using one or more markers 3a-c included therein, which are capable of at least temporarily generating a magnetic field.

In one embodiment, each medical device 1a-c only includes one marker 3a at one point and the system localizes this specific point of each medical device 1a-c. In this embodiment, the system is capable of simultaneously localizing a plurality of medical devices 1a-c. The point may correspond to a part of the medical device 1, the localization of which is of particular importance for carrying out the interventional procedure. One example of such a part is the tip of the medical device 1, when the tip has to be steered to a defined position in the patient body 2. Likewise, it may be sufficient to localize known but arbitrarily selected points of the medical devices 1.

In one particular implementation of this embodiment, which is illustrated in Fig. 1 and will be explained in more detail herein below by way of example, the medical devices 1a-c are plural brachytherapy catheters used in a HDR brachytherapy treatment. As will be further explained herein below, the positions of the markers included in these catheters se catheters comprise dwell locations for holding radiation sources and these dwell locations correspond to the parts of the catheters which are localized in the system.

In a further embodiment, the system localizes one medical device 1 which includes plural markers 3a-c at different points thereof. In this embodiment, it is possible to determine the position and orientation of the complete medical device 1. For this purpose, a virtual model of the medical device 1 may be used and fitted to the determined positions of the markers 3a-c. For this purpose, the virtual model may be positioned and oriented in a certain frame of reference in such a way that the positions of the markers 3a-c in the model correspond to the determined positions.

In one embodiment, each marker 3a-c includes a permanently magnetized material. Examples of such a material include suitable ferromagnetic or ferromagnetic materials as known to the person skilled in the art. In this embodiment, the markers 3a-c permanently generate a magnetic field, which can be measured in the system in order to determine the positions of the markers 3a-c. In this measurement, the earth magnetic field interfering with the magnetic field generated by the markers is preferably compensated for, e.g. on the basis of a calibration measurement carried out when the markers 3a-c are not included in the measurement area.

In further embodiments, the markers 3a-c only temporarily generate a magnetic field in response to a magnetic excitation. In one of these embodiments, the markers 3a-c include a magnetizable material, such as, for example, a ferromagnetic material. In a further embodiment, the markers include a non-magnetizable, electrically conductive material, such as, for example, aluminum, or a resonant circuit, particularly an LC circuit.

In order to determine the positions of markers 3a-c configured according to these embodiments, the system includes a magnetic field generator, which generates a magnetic field for exciting the markers 3a-c. This magnetic field is referred to as excitation field herein. After excitation by means of the excitation field, the markers 3a-c themselves generate a magnetic field which is measured in order to determine their positions. This magnetic field is also referred to as induced magnetic field herein.

The magnetic field generator 4 may include one or more excitation coil(s) 5, through which a controllable electrical current flows during operation in order to generate the excitation field. The excitation coil(s) 5 is/are arranged in the vicinity of a measurement area, i.e. the volume in which the part of the patient body accommodating the medical device 1 is placed during the position measurement using the system. The excitation coil(s) is/are coupled to an adjustable power source 6 of the magnetic field generator which is configured to drive the current flowing through the excitation coil(s) 5. Due to the current flowing through the excitation coil(s) 5, the excitation field is generated. In order for the excitation magnetic field to magnetize the markers 3a-c, the excitation coil 2 is arranged such that the strength of the excitation magnetic field in the entire measurement area is sufficiently high. For this purpose, a relative large single excitation coil 2 may be used. However, it is likewise possible to use multiple excitation coils 2 (which may be smaller) in order to achieve a sufficiently high magnetic field strength throughout the measurement area.

The magnetic excitation field may have an arbitrary form in the measurement area. In particular, it is not required that the magnetic excitation field is homogeneous within the area or has another particular form. Therefore, it is generally not necessary to configure the magnetic field generator 4 in a particular way in order to ensure the generation of an excitation magnetic field having a certain form. Hereby, the setup of the magnetic field generator 4 is facilitated.

For measuring the magnetic field generated by the markers 3a-c, the system further includes a plurality of magnetometers 6i. Each magnetometer 6i is configured to locally measure the strength of the magnetic field at its position. For this purpose, the magnetometers 3i may be configured in any suitable manner known to a person skilled in the art. For example, the magnetometers 6i may include hall sensors; however, other configurations are likewise possible. The magnetometers 6i may be configured as three-axis magnetometers measuring the magnetic field strength along three perpendicular directions. However, it is likewise possible to use one-axis or two-axis magnetometers measuring the magnetic field along one axis or along two perpendicular axes.

In order to determine the positions of N markers 3a-c, the magnetometers 6i are preferably capable of performing 6N or more independent measurements. In order to achieve this, the system may include at least 2N three-axis magnetometers, 3N two-axis magnetometers or 6N one-axis magnetometers. Likewise, it is possible that the system comprises a combination of magnetometers 6i measuring along different numbers of axes, when these magnetometers 6i together allow for performing at least 6N measurements.

When 6N or more independent measurements can be made, the number of measurements is equal to or larger than the number of degrees of freedom of the system of N markers 3a-c as each marker 3a-c has six degrees of freedom. Thus, there is at least one measurement value per variable included in the equations for determining the positions of the markers 3a-c so that a unique solution for the positions of all N markers 3a-c can be estimated. The six degrees of freedom of one marker 3a-c include three translational degrees of freedom (i.e. to the position within space) and three degrees of freedom for the magnetization of the marker 3a-c. These three degrees of freedom correspond to the magnetic moment of the respective seed 1i which has a magnitude and a direction in space.

The magnetometers 6i are arranged in the vicinity of the measurement area at predefined (i.e. known) positions. This does particularly mean that the magnetometers 6i are arranged at certain fixed positions with respect to a frame of reference, which is used in the localization of the medical device 1. In principle, the magnetometers 3i can be arranged at arbitrary positions in the vicinity of the measurement area as long as they are positioned sufficiently close to the measurement area to measure the strength of the magnetic field generated by the markers 3a-c.

In one exemplary implementation, the magnetometers 6i are arranged in a two-dimensional rectangular array as schematically illustrated in Fig. 2. Thus, the magnetometers 6i are arranged along straight columns and rows arranged perpendicular to the columns. In a further implementation, the magnetometers 6i may be arranged in a regular three-dimensional grid. These implementations allow for a compact arrangement of the magnetometers 6i. In particular, it is possible to include the magnetic field generator 4 and the magnetometers 6i in one relatively compact housing, which may be positioned in the vicinity of the measurement area in order to localize a medical device 1 used therein.

In further embodiments, the magnetometers 6i may arranged differently. For instance, they are included in two or more arrays of the aforementioned type and/or they are distributed around the measurement area in greater distances. Hereby, the measurement can be improved particularly in case this magnetic field is substantially homogenous in one or more limited area(s) and has a different form at more distant locations.

The magnetometers 6i and the magnetic field generator 4 are coupled to an evaluation unit 8, which is particularly configured to evaluate the magnetometer measurements to localize the medical device 1 and to control the magnetic field generator 4 (particularly the power source 6 included therein). The evaluation unit 8 may be configured as processer unit comprising a microprocessor for executing computer programs and a memory for storing the computer programs and further data. Further, the localization unit may output information about the determined position of the medical 1 for a further use. In one embodiment, it may output such information to a display device 8 in order to visualize the position of the medical device 1 in a way to be descried in more detail herein below. In addition, or as an alternative, the evaluation unit 8 may forward the information to further units which further process this information. One such further embodiment may be a planning unit 10 for generating a treatment plan for a HDR brachytherapy planning and/or adapting such a treatment plan as will be further explained below.

In order to localize the medical device 1 by means of the system, the region of the patient's body 2 including the medical device 1 is arranged within the measurement area surrounded by the magnetometers 7i and the magnetic field generator 4. In case, the markers 3a-c are not permanently magnetized, the magnetic field generator 4 is operated to excite the markers 3a-c such that they generate an induced magnetic field.

Preferably, the magnetic field generator 4 is time-varying magnetic field. In one embodiment, the magnetic field generator 4 particularly generates a magnetic field that has a periodically varying strength and preferably also has a periodically varying direction. This results in an induced magnetic field, which likewise varies periodically. In this respect, the markers are preferably configured such and the frequency of the time varying magnetic excitation field is preferably selected such that the maximum strength of the induced magnetic field or at least a significant strength of the induced magnetic field occurs at times when the strength of the magnetic excitation field is minimal, where the minimum strength of the minimum strength of the magnetic excitation field is preferably zero. This ensures, that the magnetic field in the measurement area essentially only corresponds to the induced magnetic field at time when the strength of the magnetic excitation field is minimal (zero) so that the induced magnetic field can easily be measured by means of the magnetometers 6i. In order to facilitate this measurement, the time-varying magnetic excitation field may be generated such that its strength is minimal over a certain period of time.

When markers 3a-c including a magnetizable material are exposed to the excitation magnetic field generated by the magnetic field generator 4, the magnetizable material markers magnetized so that an induced magnetic field is generated by each marker 3a-c. The induced magnet fields generated by the markers 3a-c then superimpose each other to generate an overall induced magnetic field 3a-c in the region of the measurement area.

Since the macroscopic magnetization of the magnetizable material is not built up instantaneously, the induced magnetic field is built up with a time delay with respect to the excitation magnetic field. After the macroscopic magnetization is built up, it vanishes again with a certain decay rate. When the markers 3a-c are exposed to the alternating excitation field, an induced magnetic field is thus created, which does likewise alternate with substantially the same frequency as the magnetic excitation field and with a certain delay (i.e. phase shift). The time delay for building up the induced magnetic field of the markers 3a-c particularly depends on the magnetizable material included in the markers 3a-c. As said above, the magnetizable material and the frequency of the alternating excitation magnetic field are preferably selected such that that the maximum strength of the induced magnetic field occurs when the strength of the magnetic excitation field is minimal.

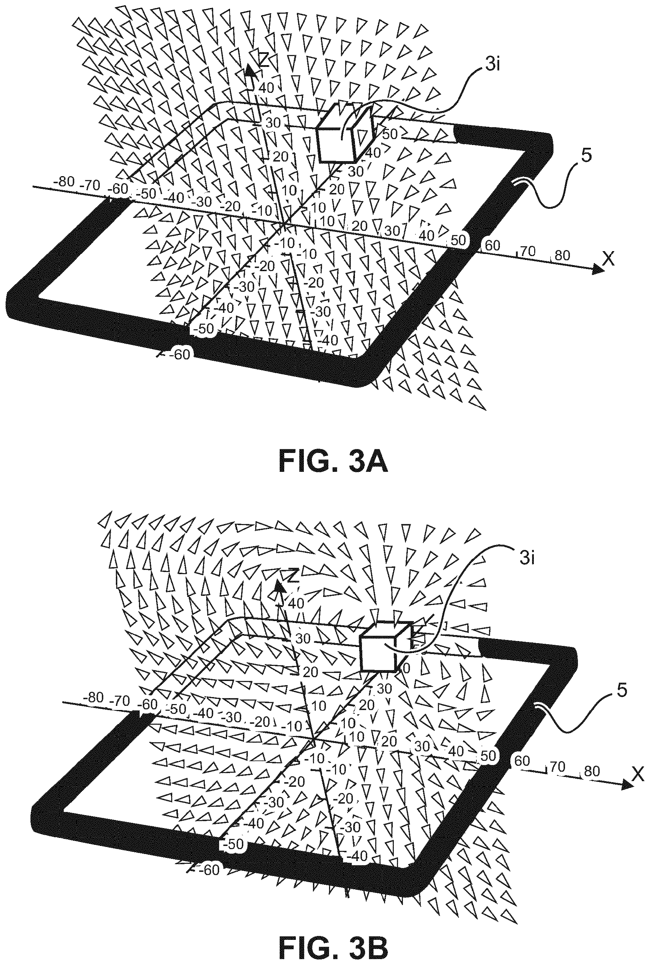

By way of example, the generation of the induced magnetic field is schematically illustrated in figures 3a and 3b for a single marker 3i (this marker may correspond to one of the markers 3a-c). Figure 3a shows the magnetic field created by a rectangular excitation coil 5 at a point in time when the magnetic excitation field has its maximum strength and when the induced magnetic field is zero. Figure 3b shows the induced magnetic field generated by the marker 3i at a later point in time, when the strength of the magnetic excitation field is zero.

When the markers 3a-c include a non-magnetizable, electrically conductive material, the time-varying magnetic excitation field induced currents (eddy currents). These currents generate a magnetic field opposing the changes in the magnetic excitation field. When the strength of the alternating excitation magnetic field drops to its minimum, the eddy currents still remain active, so that an induced magnetic field is still generated when the strength of the excitation magnetic field is minimal. Thus, the induced magnetic field can be measured by means of the magnetometers at the times when the strength of the alternating excitation magnetic field is minimal.

In case the markers 3a-c include a resonant circuit, the circuit is excited by the time varying magnetic excitation field. For this purpose, the frequency of the magnetic excitation field preferably corresponds to the resonant frequency of the resonant circuit. As a consequence, the circuit generates an induced magnetic field, which likewise varies periodically. Since there is a phase difference of 90° between the magnetic excitation field and the induced magnetic field, the strength of the induced magnetic field is maximal at the times when the strength of the magnetic excitation field is minimal (zero) so that the induced magnetic field can be measured at these times. The positions of the markers 3a-c within the patient's body are determined on the basis of the overall induced magnetic field generated by the markers 3a-c. When the magnetic field generator 4 is operated to generate a time-varying magnetic excitation field as explained above, this induced magnetic field can be measured while the strength of the magnetic excitation field is minimal. Thus, in order to measure the induced magnetic field generated by the markers 3a-c, the evaluation unit 8 reads measurement values measured by the magnetometers 7i in the time windows corresponding to the times when the strength of the excitation magnetic field is minimal (zero). In one corresponding time window, the measurements performed by the magnetometers 7i yield values for the local strength of the magnetic field at the positions at which the magnetometers 7i are located with respect to the measurement axes of the magnetometers 7i.

These values are preferably corrected with respect to the earth magnetic field interfering with the induced magnetic field during the measurements. When the markers 3a-c are essentially stationary at two consecutive measurements and when the alternating magnetic excitation field is symmetric in the half periods of its periodic course in time (i.e. when the course of the excitation in one half-period is the negative of the course in the other half-period), the induced magnetic field has the same strength and opposite directions at the end of the half periods. Thus, it is possible to determine the strength of the earth magnetic field from the sum of the values of the magnetic field measured by the magnetometers in the two consecutive measurements. As an alternative, the strength of the earth magnetic field may be determined on the basis of a calibration measured carried out, when the excitation field and the induced magnetic field are not present.

In case the markers 3a-c include a permanently magnetized material, the magnetic field generated by the markers 3a-c can be directly measured using the magnetometers 7i without having to generate an excitation magnetic field. In this case, the evaluation unit 8 reads the measurement values for the local strength of the magnetic field created by the markers 3a-c when the markers 3a-c are placed in the measurement area. These values are likewise corrected with respect to the earth magnetic field, which may be determined by means of a calibration measurement, for example.

Thus, marker the evaluation unit 8 effectively acquires from the magnetometers 7i a number of (corrected) measurement values $\vec{B}_m(\vec{x}_m)$ for the local strengths of a magnetic field generated by markers at the positions $\vec{x}_m$ of the magnetometers 7i (where not all components of $\vec{B}_m(\vec{x}_m)$ are measured in case the respective magnetometer 7i is not configured as a three-axes magnetometer). On the basis of these measurements values, the evaluation unit 8 determines the positions of the markers 3a-c. For this purpose, the evaluation unit 8 may calculate the magnetic field generated at the positions $\vec{x}_m$ by the markers based on estimated positions and orientations of the markers 3a-c in a model calculation and may determine the estimated positions and orientations of the markers 3a-c such that a difference between the calculated magnetic field and the measurement values is minimized. The model calculation is preferably carried out on the basis of the Maxwell equations. Thus, the minimization of the difference between the calculated magnetic field and the (locally) measured magnetic field corresponds to a numerically determined solution of the inverse Maxwell problem.

In the model calculation, each marker 3a-c is regarded as a magnetic dipole. Thus, the magnetic field generated by each marker 3a-c is modeled by the equation describing the magnetic field of a magnetic dipole:

$$\vec{B}_n\left(\vec{y}\right) = \frac{\mu_0}{4\pi}\left(\frac{3\left(\vec{y}-\vec{x}_n\right)\left(\vec{m}_n\cdot\left(\vec{y}-\vec{x}_n\right)\right)}{\left|\vec{y}-\vec{x}_n\right|^5} - \frac{\vec{m}_n}{\left|\vec{y}-\vec{x}_n\right|^3}\right)$$

Here, $\vec{B}_n(\vec{y})$ denotes the vector of the magnetic field (B field) generated by the marker $n$ at the position $\vec{y}$, $\vec{x}_n$ denotes the position of the marker n and $\vec{m}_n$ denotes the magnetic dipole moment of the marker $n$.

The overall magnetic field $\vec{B}_{tot}(\vec{y})$ generated by all markers 3a-c at the position $\vec{y}$ results from a superposition of the magnetic fields generated by the individual markers 3a-c and is given by $\vec{B}_{tot}(\vec{y}) = \sum_n \vec{B}_n(\vec{y})$, where the sum is calculated over all markers 3a-c.

Each magnetometer 7i measures one or more components of the field $\vec{B}_{tot}(\vec{y}_k)$ at the position $\vec{y}_k$ of the respective magnetometer 7i, and the measurements carried out by all magnetometers 7i yield a number of values $B_{tot,l}(\vec{y}_k)$, where $B_{tot,l}$ denotes the $l$-component of the field $\vec{B}_{tot}$ (i.e. the x-, y- or z-component). Thus, the measurements allow for establishing a (non-linear) system of equations in which each equation has the form

$$B_{tot,l}\left(\vec{y}_k\right) = \frac{\mu_0}{4\pi}\sum_n\left(\frac{3\left(y_l-x_{n,l}\right)\left(\vec{m}_n\cdot\left(\vec{y}-\vec{x}_n\right)\right)}{\left|\vec{y}-\vec{x}_n\right|^5} - \frac{m_{n,l}}{\left|\vec{y}-\vec{x}_n\right|^3}\right).$$

Here, the subscript $l$ again denotes the $l$-component of the respective parameter.

As unknowns, the system of equations includes (on the right-hand side) the components of the positions $\vec{x}_n$ of the markers 3a-c, which are to be estimated, and the components of the magnetic moments $\vec{m}_n$ of the markers 3a-c.

In order to estimate the positions of the markers 3a-c, the evaluation unit 8 establishes a system of equations of the aforementioned type. The system includes one equation for each value $B_{tot,l}(\vec{y}_k)$ determined on the basis of the measurements in the way described above. The sums on the right-hand side of the equations are established on the basis of the number of markers 3a-c, which is provided to the evaluation unit 8 in addition to the measured values for the magnetic field. Then, the evaluation unit 8 determines a solution of the system of equations for the unknown positions $\vec{x}_n$ of the markers 3a-c and their magnetic moments $\vec{m}_n$. Preferably, the solution of the system of equations is determined using a numerical algorithm. In this regard, any suitable algorithm known to a person skilled in the art may be applied. One example of such an algorithm is the well-known Gauss-Newton algorithm, which allows for approximating a solution of the system of equations in a least-square sense.

In such a way, the evaluation unit 8 may estimate the positions of the markers 3a-c on a (quasi-) continuous basis. For instance, it may determine one position value for each marker 3a-c for each measurement, where the measurements may be carried periodically as explained above. Thus, it is possible to monitor the positions of the markers 3a-c essentially in real time.

After having estimated the positions of the markers 3a-c, the evaluation unit 8 may provide the determined positions of the markers 3a-c for further processing thereof and/or for displaying the positions to an operator.

In both cases, the positions of the markers 3a-c may firstly be transformed into a specific frame of reference, which differs from the frame of reference in which the aforementioned evaluation is carried out by a rigid transformation (i.e. by a translation and/or rotation) and which is referred to as primary reference frame herein below. The primary reference frame may correspond to the image frame of a three-dimensional image of the patient body, which is acquired using a suitable imaging modality, such as CT imaging or MR imaging. The image shows the anatomy of the patient body 2 and, thus, the transformed positions of the markers 3a-c allow for easily determining the position of the medical device(s) 1a-c relative to the anatomy of the patient body.

The determination positions of the markers 3a-c may be displayed as such to an operator at a display unit 9. In case one medical device includes plural markers, the evaluation unit 8 may alternatively or additionally position a virtual model of the medical device in the primary reference frame in such a way that the points of the virtual model, which correspond to the points of the markers 3a-c in the real device, are positioned at the determination locations of the markers 3a-c. In such a way, the position and orientation of the virtual model be fitted to the determined locations of the markers 3a-c.

In order to display the positions of the markers 3a-c and/or the virtual model of the medical device 1 to an operator, the positions and/or the model may be overlaid over the image of the patient body and the resulting view may be presented to the operator at a display unit 9 coupled to the evaluation unit 8. Thus, it is possible for the operator to directly inspect the relative position of the markers 3a-c and/or the medical device(s) 1a-c with respect to the patient anatomy.

When the evaluation unit 8 provides the determined positions of the markers 3a-c for an automatic further processing, it may forward information about these positions to a corresponding further processing unit which may evaluate the positions in a certain manner. In one implementation, the further processing unit may compare the determined positions with desired positions of the medical device(s) 1a-c in order to determine deviations between the determined and desired positions. On the basis of these deviations, further actions may then be taken. For instance, the medical device(s) 1a-c may be repositioned such that it/they is/are arranged at the desired position(s).

In one related implementation, the positions of brachytherapy catheters 1a-c are determined in the system and the determined positions are compared with desired positions of the catheters 1a-c prescribed for the brachytherapy treatment. This implementation will be now explained in more detail:

In a HDR brachytherapy treatment, the catheters 1a-c are used for delivering radiation sources, which may be configured as radioactive particles, to a target region including the target structure of the treatment, such as a tumor. By means of the catheters 1a-c, the radiation sources can be delivered to the target region and hold at defined positions, which are also referred to as dwell positions herein, for defined time periods, which are also referred to as dwell times. In the embodiment illustrated in Fig. 1, the radiation sources are automatically delivered through the catheters 1a-c to the dwell locations 11a-c from an afterloader device 12 (in Fig. 1, dwell positions at the distal ends of the catheters 1a-c are shown by way of example).

In the present implementation, the localization system may be enabled to localize the catheters 1a-c within the patient body 2. For this purpose, each catheter 1a-c is provided with a marker 3a-c at a certain position thereof and the evaluation unit 8 localizes the markers 3a-c of the catheters 1a-c as explained above. The markers 3a-c may particularly be arranged in the vicinity of the catheter tips. However, other arrangements of the markers 3a-c are likewise possible.

The brachytherapy treatment is delivered in accordance with a treatment plan, which specifies the relevant treatment parameters including the dwell positions and the dwell times. The treatment plan is generated in a planning unit 10 on the basis of a three-dimensional planning image of the region of the patient body 2, which shows the target structure and surrounding organs, which are also referred to as organs at risk (OARs). The frame of the planning image may also serve as the primary reference frame with respect to the localization of the catheters 1a-c. Thus, the evaluation unit may transform determined position information into the frame of the planning image. In the planning unit 10, the treatment plan is determined such that a prescribed radiation dose is delivered to the target structure and that the surrounding OARs receive a radiation dose, which is as low as possible and does particularly not exceed prescribed limits.

In order to generate the treatment plan in such a way, target dwell positions may be determined at first on the basis of the positions of the target structure and the OARs by applying a heuristic determination procedure. Known examples of such a procedure include a k-means clustering procedure and a centroidal voronoi tessellation. On the basis of the dwell positions, the dwell times may then be determined in order to achieve an optimized dose distribution in which the prescribed is delivered to the target structure and the dose limits for the OARs are met. The determination may be made using a suitable optimization procedure as known to the person skilled in the art as such. For instance, the dose goals for the target structure and the dose limits for the OARs may be translated into a cost function which depends on the dwell times, and the planning unit 10 may minimize this cost function in an automatic or user-assisted iterative optimization procedure to determine the dwell times to be applied in the treatment of the patient.

In one embodiment, the dwell times are determined based on the target dwell positions and, thus, the target dwell positions correspond to the planned dwell positions which should be used in the treatment. In this case, the catheters 1a-c may be positioned in the patient body 2 in accordance with planned catheter positions corresponding to the target dwell positions upon having generated the complete treatment plan. Here, the correct positioning of the catheters may be guided by means of images acquired using a suitable imaging modality, such as, for example computed tomography (CT) imaging. As an alternative, the catheters 1a-c may be inserted in the patient body in accordance with the target dwell positions before the dwell times are determined. Then, the actual positions and orientations of the catheters 1a-c may be determined (e.g. in the basis of an image), and the dwell times may be calculated based on the determined positions.

When the catheters 1a-c are positioned for carrying out the treatment and when the treatment plan has been generated, but prior to the delivery of the radiation sources to the dwell locations, and/or during the treatment (i.e. when the radiation sources are held at the dwell positions), the evaluation unit 8 preferably monitors the positions of the catheters 1a-c by determining the positions of the markers 3a-c as explained above. The determined locations may be transmitted to the planning unit 10 and the planning unit 10 may check whether the determined positions of the catheters 1a-c correspond to the planned catheter positions. More specifically, the planning unit 10 determines planned positions of the markers 3a-c on the basis of the planned catheter positions and checks whether the actual positions of the markers 3a-c deviate from the planned positions.

In one related embodiment, the evaluation unit 10 may determine absolute values of the positions of the markers 3a-c in a certain reference frame. These absolute values can be computed based on the measured positions of the markers 3a-c and based on the known positions of the magnetometers 6i in the reference frame. The planning unit 10 then compares the absolute values of the positions of the markers 3a-c with the values of the planned positions in the same reference frame. If the measured absolute positions of the markers do not correspond to the planned positions, the planning unit 10 determines a deviation between the actual and the planned positions of the catheters.

In a further embodiment, the planning unit 10 computes planned relative positions between the markers 3a-c based on their planned positions. Further, the planning unit 10 determines the actual relative positions between the markers 3a-c based on the measured positions of the markers 3a-c and compares these relative positions with the planned relative positions. If the relative positions of the markers 3a-c determined on the basis of the measured positions of the markers 3a-c do not correspond to the planned relative positions, the planning unit 10 determines a deviation between the actual and the planned positions of the catheters 1a-c. The determined relative positions between the markers may comprise the relative positions between one or more of the markers 3a-c and each further marker 3a-c.

Compared with the embodiment, in which the absolute positions of the markers 3a-c are evaluated, the latter embodiment has the advantage that no absolute position reference is necessary. In the latter embodiment, it is not possible to detect a uniform displacement of all catheters with respect to the desired positions. However, it will be appreciated that such uniform displacement is very unlikely.

In case the planning unit 10 determines a deviation between the actual and the planned positions of the catheters, an alarm routine may be initiated. This routine may comprise a notification of the planner of the brachytherapy treatment and/or and an automatic stop of the brachytherapy treatment. When the planner is notified, he/she may decide how to proceed with the treatment. For instance, it may be possible to continue the treatment in case the deviations between the actual and the planned positions are smaller. Or it may be necessary to re-plan the brachytherapy treatment and/or to completely re-initiate the treatment.

Thus, the measurement of the positions of the markers allows for a quality assurance during a brachytherapy treatment of a patient, which can easily be carried out using a system as described above.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

**Claims**

1.  A system for localizing at least one medical device (1a-c) in a patient body (2), the at least one medical device (1a-c) comprising at least one marker (3a-c) which is capable of at least temporarily generating a magnetic field, the system comprising

    - magnetometers (7i) arranged outside the patient body (2) for measuring the magnetic field generated by the at least one marker (3a-c), and
    - an evaluation unit (8) coupled to the magnetometers (7i) and configured to determine the position of the at least one medical device (1a-c) on the basis of the magnetic field measurement by the magnetometers (7i).

2.  The system as defined in claim 1, configured for simultaneously localizing a plurality of medical devices (1a-c), wherein each of the plurality of medical devices (1a-c) comprises one marker (3a-c) at a specific point thereof.

3.  The system as defined in claim 1, configured for localizing one medical device, wherein the medical device comprises a plurality of markers and wherein the evaluation unit is configured to determine the positions of the medical device by determining positions of the markers and fitting a model of the medical device to the determined positions.

4.  The system as defined in claim 1, wherein the at least one marker (3a-c) is configured to generate a magnetic field in response to a magnetic excitation by a magnetic excitation field.

5.  The system as defined in claim 4, wherein the at least one marker (3a-c) comprises a magnetizable material and wherein the magnetic field is generated as a result of a magnetization of the magnetizable material by means of the magnetic excitation.

6.  The system as defined in claim 4, wherein the at least one marker (3a-c) comprises an electrically conductive material or a resonant circuit and wherein the magnetic field is generated as a result of an excitation by a time-varying magnetic excitation field.

7.  The system as defined in claim 4, further comprising at least one magnetic field generator (4) for generating the magnetic excitation field.

8.  The system as defined in claim 7, wherein the magnetic field generator (4) is configured to generate the magnetic excitation field with a varying strength and wherein the magnetic field measurement is carried out at a time when the strength of the magnetic excitation field is minimal.

9.  The system as defined in claim 1, wherein the evaluation unit (8) is configured to calculate values of the magnetic field generated by the markers (3a-c) at the locations of the magnetometers (7i) on the basis of estimated positions of the markers (3a-c) in a model calculation and to determine the positions of the markers (3a-c) by at least approximately minimizing a difference between the calculated values of the magnetic field and values measured by the magnetometers (7i).

10. The system as defined in claim 2, wherein the medical devices (1a-c) are brachytherapy catheters for holding radiation sources (11a-c) during a brachytherapy treatment, each brachytherapy catheter comprising one marker (3a-c).

11. The system as defined in claim 10, further comprising a planning unit (10) configured to detect a deviation between the determined positions of the markers (3a-c) and desired positions thereof, which correspond to desired positions of the brachytherapy catheters during the brachytherapy treatment.

12. The system as defined in claim 11, wherein the planning unit (10) is configured to compare relative positions of the markers (3a-c) computed based on the determined positions of the markers (3a-c) with desired relative positions of the markers (3a-c) determined based on the desired positions of the markers and to detect a deviation between the determined positions of the markers (3a-c) and desired positions thereof, if it determines that at least one of the relative positions of the markers (3a-c) deviates from a corresponding desired relative position.

13. The system as defined in claim 11, wherein the planning unit (10) is configured to initiate an alarm routine when it detects a deviation between the determined positions of the markers (3a-c) and the desired positions thereof.

**14.** A method for localizing at least one medical device (1a-c) in a patient body (2), the at least one medical device (1a-c) comprising at least one marker which is capable of at least temporarily generating a magnetic field, the method comprising

- receiving measurement information from magnetometers (7i) arranged outside the patient body (2), the measurement information relating to measurements of the magnetic field generated by the at least one marker, and
- determining the position of the at least one medical device (1a-c) on the basis of the measurement information.

**15.** A computer program comprising program code for instructing a computer device to perform a method as defined in claim 14 when the program code is executed in the computer device.

**FIG. 1**

**FIG. 2**

## FIG. 3A

## FIG. 3B

Europäisches
Patentamt

European
Patent Office

Office européen
des brevets

# EUROPEAN SEARCH REPORT

Application Number

EP 18 16 4826

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2016/193185 A1 (KONINKLIJKE PHILIPS NV [NL]) 8 December 2016 (2016-12-08) | 1,2,10, 14,15 | INV. A61B34/10 A61B34/20 |
| Y | * figure 1 * * claim 14 * * page 4, lines 32, 33 * * page 5, lines 1, 2, 5-7 * * page 6, lines 21-23 * * page 7, lines 17,18 * * page 9, lines 18-20 * * page 11, line 34 - page 12, line 6 * * page 17, lines 8-13 * ----- | 11-13 | ADD. A61N5/10 A61B90/00 |
| X | US 2009/299174 A1 (WRIGHT J NELSON [US] ET AL) 3 December 2009 (2009-12-03) * figures 1, 4A, 4D, 4E * * claims 25, 52 * * paragraphs [0041], [0055] - [0058], [0064] - [0066] * ----- | 1,14,15 | |
| Y | WO 2006/012631 A2 (CALYPSO MED TECHNOLOGIES INC [US]; MEIER ERIC [US]; MATE TIMOTHY P [US]) 2 February 2006 (2006-02-02) * paragraph [0061] * ----- | 11-13 | TECHNICAL FIELDS SEARCHED (IPC) A61B A61N |

~~The present search report has been drawn up for all claims~~

| Place of search | Date of completion of the search | Examiner |
|---|---|---|
| Munich | 28 September 2018 | Schleich, Florian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

2, 10-13(completely); 1, 14, 15(partially)

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

Application Number

EP 18 16 4826

The Search Division considers that the present European patent application does not comply with the requirements of unity of invention and relates to several inventions or groups of inventions, namely:

```
1. claims: 2, 10-13(completely); 1, 14, 15(partially)

    A system for simultaneously localizing a plurality of
    medical devices, wherein each of the plurality of medical
    devices comprises one marker at a specific point thereof.
                        ---

2. claims: 3(completely); 1, 14, 15(partially)

    A system for localizing one medical device, wherein the
    medical device comprises a plurality of markers and wherein
    the evaluation unit is configured to determine the positions
    of the medical device by determining positions of the
    markers and fitting a model of the medical device to the
    determined positions.
                        ---

3. claims: 4-8(completely); 1, 14, 15(partially)

    A system for localizing at least one medical device, the at
    least one medical device comprising at least one marker,
    wherein the at least one marker is configured to generate a
    magnetic field in response to a magnetic excitation by a
    magnetic excitation field.
                        ---

4. claims: 9(completely); 1, 14, 15(partially)

    A system for localizing at least one medical device, wherein
    the evaluation unit is configured to calculate values of the
    magnetic field generated by the markers at the locations of
    the magnetometers on the basis of estimated positions of the
    markers in a model calculation and to determine the
    positions of the markers by at least approximately
    minimizing a difference between the calculated values of the
    magnetic field and values measured by the magnetometers.
                        ---
```

## ANNEX TO THE EUROPEAN SEARCH REPORT
## ON EUROPEAN PATENT APPLICATION NO.

EP 18 16 4826

This annex lists the patent family members relating to the patent documents cited in the above-mentioned European search report.
The members are as contained in the European Patent Office EDP file on
The European Patent Office is in no way liable for these particulars which are merely given for the purpose of information.

28-09-2018

| Patent document cited in search report | | Publication date | Patent family member(s) | | Publication date |
|---|---|---|---|---|---|
| WO 2016193185 | A1 | 08-12-2016 | CN | 107708804 A | 16-02-2018 |
| | | | EP | 3302700 A1 | 11-04-2018 |
| | | | JP | 2018516129 A | 21-06-2018 |
| | | | US | 2018140869 A1 | 24-05-2018 |
| | | | WO | 2016193185 A1 | 08-12-2016 |
| US 2009299174 | A1 | 03-12-2009 | US | 2009299174 A1 | 03-12-2009 |
| | | | WO | 2005067563 A2 | 28-07-2005 |
| WO 2006012631 | A2 | 02-02-2006 | EP | 1778357 A2 | 02-05-2007 |
| | | | JP | 2008507367 A | 13-03-2008 |
| | | | US | 2006058648 A1 | 16-03-2006 |
| | | | US | 2006074301 A1 | 06-04-2006 |
| | | | US | 2006074302 A1 | 06-04-2006 |
| | | | US | 2013172657 A1 | 04-07-2013 |
| | | | WO | 2006012631 A2 | 02-02-2006 |

EPO FORM P0459

For more details about this annex : see Official Journal of the European Patent Office, No. 12/82